# EUROPEAN PATENT SPECIFICATION

(11) **EP 0 993 579 B1**
(45) Date of publication and mention of the grant of the patent: **05.09.2007**
(21) Application number: 98933012.1
(22) Date of filing: 02.07.1998
(51) Int. Cl.: F21V 8/00

(54) **LIGHT DELIVERY SYSTEM AND APPLICATIONS THEREOF**
BELEUCHTUNGSSYSTEM UND DESSEN ANWENDUNG
SYSTEME SERVANT A APPORTER DE LA LUMIERE ET APPLICATIONS DE CE DERNIER

(30) Priority: 02.07.1997 US 886666
(43) Date of publication of application: 19.04.2000
(73) Proprietor: LUMITEX, INC., Strongsville, OH 44136 (US)
(72) Inventor: PARKER, Jeffery, Strongsville, OH 44136 (US); WILLIAMS, Jeffrey, Ravenna, OH 44266 (US); MILLER, Mark, Parma, OH 44134 (US)
(74) Representative: McCallum, Graeme David
(86) International application number: PCT/US1998/013628
(87) International publication number: WO 1999/001696

(56) References cited:
- WO-A-96/17206
- WO-A-96/37730
- DE-A- 4 234 050
- DE-U- 8 600 868
- DE-U- 8 607 483
- US-A- 5 005 108
- US-A- 5 281 134

## Description

### Field of Invention

The present invention relates to applications of a light delivery system as a portion of a surgical instrument.

### Background to the Invention

While lighting delivery systems are generally known, prior art systems are not easily adapted for use in connection with functional instruments, such as surgical instruments. In this respect, current light delivery systems do not provide optimal illumination for a variety of desired lighting conditions. In addition, current light delivery systems are not suitable to form a structural component of an instrument. Moreover, current light delivery systems are costly to replace, and are thus not well suited for conditions which necessitate that the light delivery system be disposable, such as where sterilization is required.

DE-A1-4234050 discloses an instrument for inspecting the interior of a nostril as shown in Figure 1 thereof, and which may be used for aiding a surgical procedure there. The instrument comprises a handle and a blade portion connected to the handle for opening the nostril. A light emitter extends through the handle and blade portion so that it emits light only from the end of the emitter where it protrudes from the instrument.

DE-U1-8600868 discloses a microsurgical forceps for aiding a surgical procedure. The forceps comprise a handle and a blade portion connected to the handle. This document discloses the light emitter extending through a tube and again light is only emitted from the end of the tube.

In these prior art documents, light is only emitted from the end of the light emitter which limits the level of illumination which can be achieved. The present invention overcomes these and other disadvantages of prior art light delivery systems.

### Summary of the Invention

Accordingly to the present invention there is provided an instrument for aiding a surgical procedure comprising a handle and a retractor arm having one end connected to the handle and another end remote from the one end, characterized by an elongated light emitter extending along the length of the retractor arm, and a connector for connecting the light emitter to a light distributor, the light emitter having light extracting formations along at least a portion of the length of the light emitter.

The arrangement according to the present invention enables a higher level of illumination to be achieved.

In a preferred embodiment of the present invention, the light emitter may be substantially transparent. Also the light emitter may be oriented at an angle with respect to the handle. In a further preferred embodiment the retractor arm may be oriented at an angle with respect to the handle. The retractor arm may include a generally curved cross-section.

The light extracting formations may comprise a printed pattern on a surface of the light emitter. It is also preferred that the light extracting formations are along substantially the entire length of the light emitter.

Also attachment means may be provided for attaching the light emitter to the retractor arm such that the light emitter and the retractor arm are substantially parallel. The attachment means may comprise for example clips.

In a preferred embodiment the light distributor may comprise an optic light guide cable. This cable may be rigid or flexible.

Still other advantages of the invention will become apparent to those skilled in the art upon a reading and understanding of the following detailed description, accompanying drawings and appended claims.

### Brief Description of the Drawings

The invention may take physical form in certain parts and arrangements of parts, a preferred embodiment and method of which will be described in detail in this specification and illustrated in the accompanying drawings which form a part hereof, and wherein:
Fig. 1 is an enlarged perspective view of a portion of the light emitter shown in Fig. 4A;
Fig. 2 is an enlarged transverse section through the light emitter shown in Fig. 1;
Fig. 3A is an enlarged plan view of a portion of a light emitter, showing one form of pattern of light extracting deformities on the light emitter;
Fig. 3B-3D are enlarged schematic perspective views of a portion of a light emitter showing other forms of light extracting deformities formed in or on the light emitter;
Fig. 4A is a perspective view of a light delivery system, wherein the light delivery system is attachable to a suction/blower device;
Fig. 4B is a perspective view of the light delivery system shown in Fig. 4A, as attached to the suction/blower device;
Fig. 4C is a perspective view of an alternative embodiment of the attachment means for the light delivery system;
Fig. 5A is a perspective view of a suction/blower device having an integrated light delivery system;
Fig. 5B is an enlarged cross-sectional view taken along line 5B-5B of Fig. 5A;
Fig. 5C is an alternative embodiment of the cross-sectional view taken along line 5B-5B of Fig. 5A;
Fig. 6 is a perspective view of another type of suction/blower device having an integrated light delivery system;
Fig. 7 is a perspective view of yet another type of suction/blower device having an integrated light delivery system;
Fig. 8 is a perspective view of an electrosurgical pencil including the light delivery system of the present invention;
Fig. 9A is a perspective view of a transillumination tray including the light delivery system of the present invention;
Fig. 9B is a cross-sectional view taken along line 9B-9B of Fig. 9A, with a vein/artery located in the transillumination tray;
Fig. 10A is a perspective view of a stabilizer including an integrated light delivery system;
Fig. 10B is a side view of the stabilizer shown in Fig. 10A;
Fig. 11 is a perspective view of a plurality of retractors including a light delivery system;
Fig. 12 is a top view of a forceps including an integrated light delivery system;
Fig. 13 is a perspective view of a multi-purpose lighting device including a light delivery system;
Fig. 14 is a sectional view of the multi-purpose lighting device taken along line 14-14 of Fig. 13;
Fig. 15A is a perspective view of a lighting device including a light delivery system;
Fig. 15B is a sectional view of the lighting device taken along line 15-15 of Fig. 15A.
Fig. 16A is a perspective view of a "rope" lighting device;
Fig. 16B is a cross-sectional view of the lighting device taken along line 16-16 of Fig. 16A;
Fig. 17 is a top view of a trans-illuminating forceps including an attachable light delivery system;
Fig. 18 is a perspective view of a trans-illuminating retractor including an attachable light delivery system;
Fig. 19A is a perspective view of a spring-formed "rope" lighting device;
Fig. 19B is a cross-sectional view of the lighting device taken along line 19-19 of Fig. 19A;
Fig. 20A is a perspective view of a smoke evacuation tube having an integrated light delivery system;
Fig. 20B is a cross-sectional view of the smoke evacuation tube taken along line 20-20 of Fig. 20A;
Fig. 21A is a perspective view of a suction tube having an integrated light delivery system;
Fig. 21B is a cross-sectional view of the suction tube taken along line 21-21 of Fig. 21A;
Fig. 22A is a perspective view of a suction tube having an attachable light delivery system;
Fig. 22B is a cross-sectional view of the suction tube taken along line 22-22 of Fig.22A;
Fig. 23A is a perspective view of a ring-shaped "rope" lighting device; and
Fig. 23B is a cross-sectional view of the lighting device taken along line 23-23 of Fig. 23A.

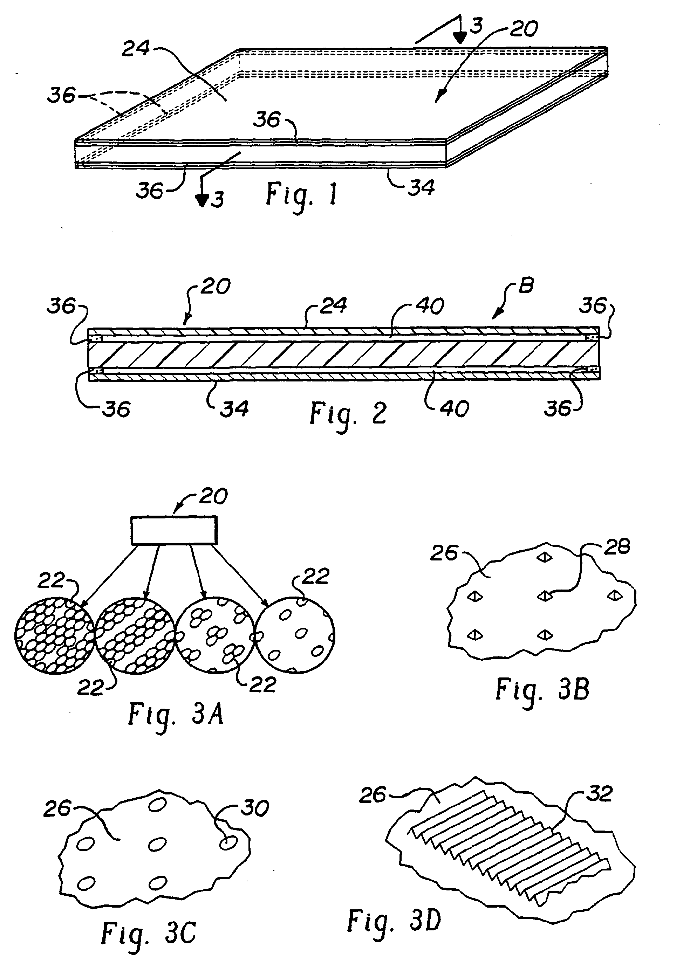

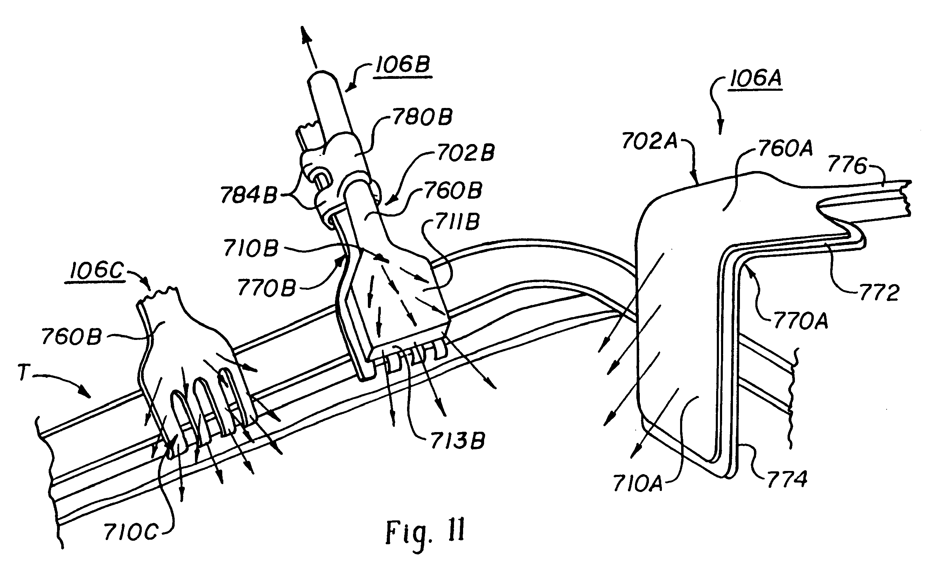

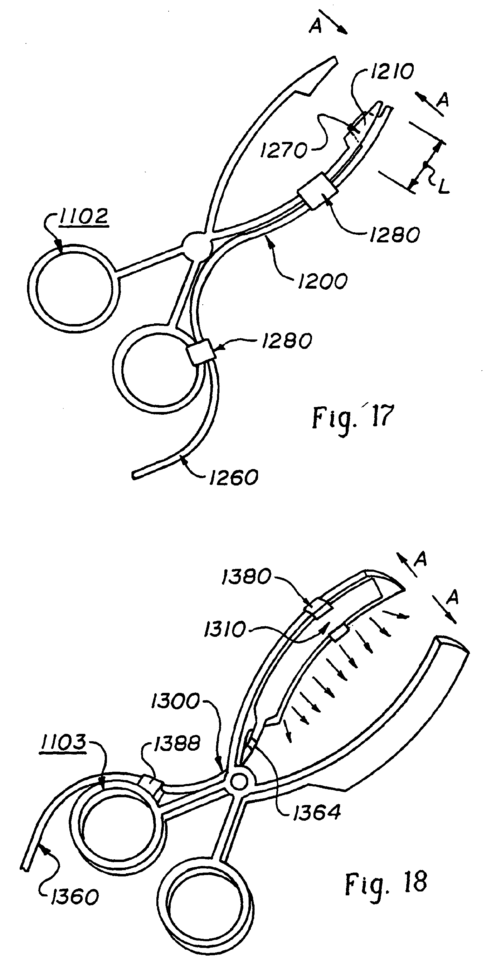

Figures 1 to 10, 12 to 17 and 19 to 23B are presented as examples useful for understanding the present invention, embodiments of which are shown in figures 11 and 18.

### Detailed Description of the Preferred Embodiment

Referring now to the drawings wherein the showings are for the purposes of illustrating a preferred embodiment of the invention only and not for purposes of limiting same,

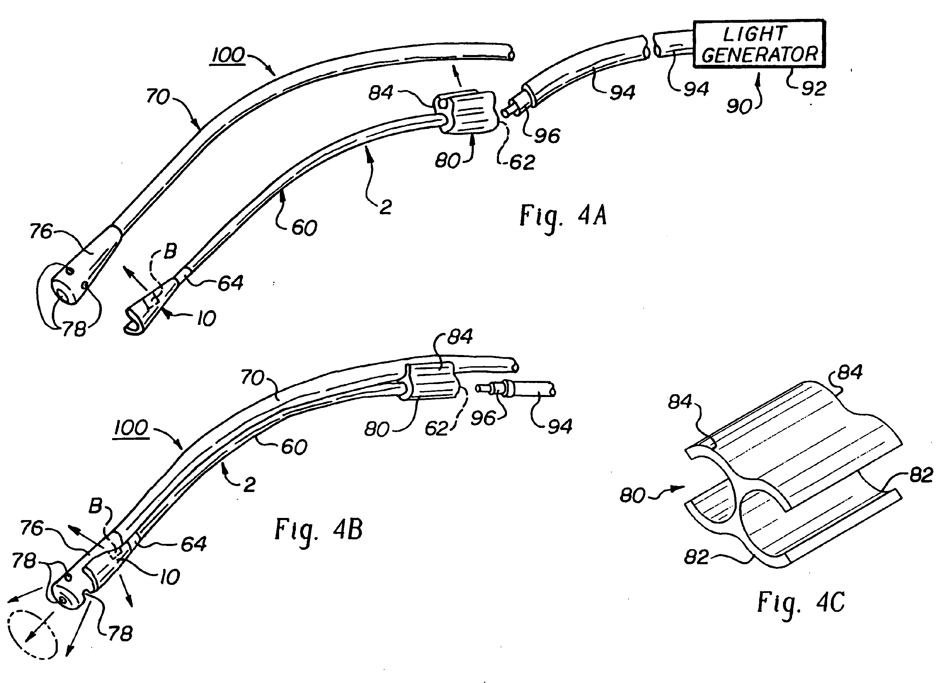

Figs. 4A and 4B illustrate a suction/blower device 100 having an externally mounted light delivery system 2. Fig. 4A shows a light delivery system 2 detached from suction/blower device 100, while Fig. 4B shows light delivery system 2 attached to suction/blower device 100. It should be appreciated that device 100 can take many forms including a surgical instrument, as will be illustrated below.

Light delivery system 2 is generally comprised of a light emitter 10, a light distributor 60, and an attachment means 80. Light emitter 10 focuses light of varying intensity in a predetermined direction or pattern. As a result, an associated viewing field is illuminated with a predetermined light characteristic. Light distributor 60 (e.g., optic light pipe) transmits light from a light source 90 to light emitter 10. Attachment means 80 provides a support structure for coupling light delivery system 2 to device 100. In this regard, attachment means 80 may include tabs, hooks or the like.

Light emitter 10 is comprised of a transparent or translucent light emitting material of any suitable type, including acrylic, polycarbonate, glass, epoxy, resins or the like. Emitter 10 may be substantially flat, suitably curved, may be formed of single or multiple layers, and may have different thicknesses and shapes. Moreover, emitter 10 may be flexible, or rigid, and may be made out of a variety of compounds. It should also be appreciated that emitter 10 may be hollow, filled with liquid, air, or be solid, and may have holes or ridges formed therein.

Means for directing light in desired directions and patterns, and providing various light intensity levels will now be described with reference to Figs. 1 and 2, which show a section B of light emitter 10. Light extracting formations including deformities, disruptions or lenses may be provided on one or more selected light surface areas 20 on one or more sides 21 or edges 23 of emitter 10. As used herein, the term light extracting formation is to mean any change in the shape or geometry of the surface and/or coating or surface treatment that causes a portion of the light to be emitted. Fig. 3A schematically shows one such light surface area 20 on which a pattern of light extracting deformities or disruptions 22 is provided. The pattern of light extracting deformities or disruptions 22 shown in Fig. 3A includes a variable pattern which breaks up the light rays such that the internal angle of reflection of a portion of the light rays will be great enough to cause the light rays either to be emitted out of emitter 10 through the side or sides on which the light extracting deformities or disruptions 22 are provided or reflected back through the emitter 10 and emitted out the other side thereof.

Light extracting formations can be produced in a variety of manners, for example, by providing a painted pattern, an etched pattern, a machined pattern, a printed pattern, a hot stamped pattern, a molded pattern, a curved surface (i.e., lens), a diffraction grating, a prismatic surface or the like on selected light surface areas 20 of emitter 10. An ink or printed pattern may be applied for example by pad printing, silk screening, ink jet, heat transfer film process or the like. The deformities or disruptions may also be printed on a sheet or film which is used to apply the deformities or disruptions to light surface area 20. This sheet or film may become a permanent part of emitter 10 for example by attaching or otherwise positioning the sheet or film against one or both sides of the emitter light surface area similar to the sheet or film 24 shown in Figs. 1 and 2 in order to produce a desired effect.

By varying the density, opaqueness or translucence, shape, depth, color, area, index of refraction, diffraction grating, or type of light extracting formations, the light output of emitter 10 can be controlled. The light extracting formations may be used to control the direction and/or percent of light emitted from any area of emitter 10. For instance, less and/or smaller size deformities 22 may be placed on emitter 10 in areas where less light output is wanted. Conversely, a greater percentage of and/or larger deformities 22 may be placed on emitter 10 in areas where greater light output is desired.

Varying the percentages and/or size of deformities 22 in different areas of emitter 10 is necessary in order to provide a uniform light output distribution. For example, the amount of light traveling through light emitter 10 will ordinarily be greater in areas closer to the light source than in other areas further removed from the light source. A pattern of light extracting deformities 22 may be used to adjust the light variances within the emitter, for example, by providing a denser concentration of light extracting deformities with increased distance from the light source thereby resulting in a more uniform light output distribution from light emitter 10. The deformities 22 may also be used to control the output ray angle distribution of the emitted light to suit a particular application.

It should be appreciated that other light extracting formations are suitably provided in addition to or in lieu of the patterns of light extracting deformities 22 shown in Fig. 3A. As indicated above, other light extracting formations include lenses, prismatic surfaces, depressions or raised surfaces of various shapes using more complex shapes in a mold pattern may be molded, etched, stamped, thermoformed, hot stamped or the like into or on one or more surface areas (e.g., sides and edges) of the light emitter. Lenses (e.g., pillow lenses) can be used to provide diffuse light (by spreading light rays) and directional light (by focusing light rays). Figs. 3B and 3C show areas 26 on which prismatic surfaces 28 or depressions 30 are formed in the emitter surface area, whereas Fig. 3D shows prismatic or other reflective or refractive surfaces 32 formed on the exterior of the emitter surface area. The prismatic surfaces, depressions or raised surfaces will cause a portion of the light rays contacted thereby to be emitted from the light emitter. Also, the angles of the prisms, depressions or other surfaces may be varied to direct the light in different directions to produce a desired light output distribution or effect, or to project a spot image or pattern of light to a specific area or region. Moreover, the reflective or refractive surfaces may have shapes or a pattern with no specific angles to reduce moire or other interference effects. In addition, the light rays emitted from the emitter may provide generally shadow less or homogenous light. In this regard, the emitter may simultaneously illuminate a 3-D object from a plurality of sides.

As best seen in the cross-sectional view of Fig.2, a back reflector 34 (including trans reflectors) may be attached or positioned against one side of the panel member 14 of Fig. 1 using a suitable adhesive 36 or other method in order to improve light output efficiency of light emitter 10 by reflecting the light emitted from that side back through the panel for emission through the opposite side. Additionally, a pattern of light extracting deformities 22, 28, 30 and/or 32 may be provided on one or both sides of the light emitter in order to change the path of the light so that the internal critical angle is exceeded and a portion of the light is emitted from one or both sides of the light emitter. Moreover, a transparent film, sheet or plate member 24 may be attached or positioned against the side or sides of the emitter from which light is emitted using a suitable adhesive 36 or other method in order to produce a desired effect.

Member 24 may be used to further improve the uniformity of the light output distribution. For example, member 24 may be a colored film, a diffuser, or a label or display, a portion of which may be a transparent overlay that may be colored and/or have text or an image thereon.

If adhesive 36 is used to adhere the back reflector 34 and/or film 24 to the emitter, the adhesive is preferably applied only along the side edges of the emitter, and if desired the end edge opposite light transition areas, but not over the entire surface area or areas of the emitter because of the difficulty in consistently applying a uniform coating of adhesive to the panel. Also, the adhesive changes the internal critical angle of the light in a less controllable manner than the air gaps 40 (see Fig. 2) which are formed between the respective surfaces of the emitter and the back reflector 34 and/or member 24 when only adhered along the peripheral edges. Additionally, longer emitters are achievable when air gaps 40 are used. If adhesive were to be used over the entire surface, the pattern of deformities could be adjusted to account for the additional attenuation in the light caused by the adhesive.

The light emitter disclosed herein may be used for a great many different applications including for example LCD back lighting or lighting in general, decorative and display lighting, automotive lighting, dental lighting, phototherapy, photodynamic therapy, or other medical lighting, membrane switch lighting, and sporting goods and apparel lighting or the like. Also the emitter may be formed such that the deformities are transparent without a back reflector. This allows the emitter to be used such that the application is viewed through the transparent emitter.

The light that is transmitted by light distributor 60 to light emitter 10 may be emitted along the entire length of light emitter 10 or from one or more light output areas along the length of the panel as desired to produce a desired light output distribution to fit a particular application.

Light distributor 60 is a formed light conduit adapted to propagate light therethrough via internal reflection. In the embodiment illustrated in Fig. 4A and 4B, light distributor 60 takes the form of an optic light pipe. Light distributor 60 includes an interface 64 and a connecting member 62. Interface 64 interfaces light distributor 60 with light emitter 10. Connecting member 62 facilitates connection of light distributor 60 with light source 90 (described below). It should be appreciated that light distributor 60, light emitter 64, and light source 90 may be formed as one unitary member without interface 64 and connecting member 62.

Light source 90 may take many forms as will be discussed below. In the embodiment of the present invention shown in Figs. 4A and 4B, light source 90 is generally comprised of a generator 92 and a cable 94. Generator 92 may be, for example, a 300 Watt Xenon light source. Cable 94 includes a connecting member 96, which mates with connecting member 62 of light distributor 60.

It should be appreciated that light source 90 illustrated in Figs. 4A and 4B is shown solely for the purpose of illustrating an embodiment of the present invention. In this respect, light source 90 may also be of other suitable types including, an arc lamp, an incandescent bulb (which also may be colored, filtered or painted), a lens end bulb, a line light, a halogen lamp, a light emitting diode (LED), a chip from an LED, a neon bulb, a fluorescent tube; a laser or laser diode, or any other suitable light source. For example, light source 90 may take the form of any of the types disclosed in U.S. Patent Nos. 4,897,771 and 5,005,108, assigned to the same assignee as the present application. Additionally, the light source may be a multiple colored LED, or a combination of multiple colored radiation sources in order to provide a desired colored or white light output distribution. For example, a plurality of colored lights such as LEDs of different colors (red, blue, green) or a single LED emitting a selected spectrum may be employed to create white light or any other colored light output distribution by varying the intensities of each individual colored light.

Attachment means 80 is suitably molded as an integral part of light distributor 60 (Fig. 4A), attaches to both the light distributor and the associated device (Fig. 4C), or forms a part of device 100. In the embodiment shown in Fig. 4A and 4B, attachment means 80 is fixed to light distributor 60, wherein gripping means 84 are provided for attaching light delivery system 2 to device 100. Attachment means 80 allows light delivery system 2 to be easily and conveniently attached to and detached from suction/blower device 100. As a result, light delivery system 2 is easily replaced where sterilization is required.

In the embodiment shown in Fig. 4C, one form of attachment means 80 includes engagement means 82 and 84 for fixing light delivery system 2 to a device. In this respect, engagement means 82 are engagable with light distributor 60, while engagement means 84 are engagable with a portion of the device. It should be appreciated that engagement means 82 and/or engagement means 84 are suitably integral with light distributor 60 and the device, respectively. However, in the case where convenient replacement of light delivery system 2 is desired (e.g., when sterilization is required) engagement means 82 and/or engagement means 84 will preferably provide for convenient removal of light delivery system 2 from the device. For instance, in the embodiment shown in Figs. 4A and 4B, engagement means 84 takes the form of a clamp, which allows for simple attachment and detachment of light delivery system 2 from device 100. It should be appreciated that engagement means 82 and 84 may take the form of other suitable fastening members including cables, snaps, clips, tabs, adhesives, and the like.

Device 100 includes a tube 70 having a tip portion 76. Tip portion 76 is comprised of a plurality of openings 78, which are in communication with tube 70. Light emitter 10 is suitably dimensioned to receive tip portion 76, when light delivery system 2 is attached to device 100 (Fig. 4B). It should be noted that light emitter 10 is suitably formed to provide diffuse light in directions transverse to the longitudinal axis of device tip portion 76, and to provide direct light in a direction generally parallel to the longitudinal axis of tip portion 76. As indicated above, the direct light provides maximum illumination on the material being suctioned or blown. At the same time, the diffuse light provides sufficient, but not over bright, illumination of the area surrounding the material being suctioned or blown. As a result, the user's vision of the material being suctioned or blown is not impaired.

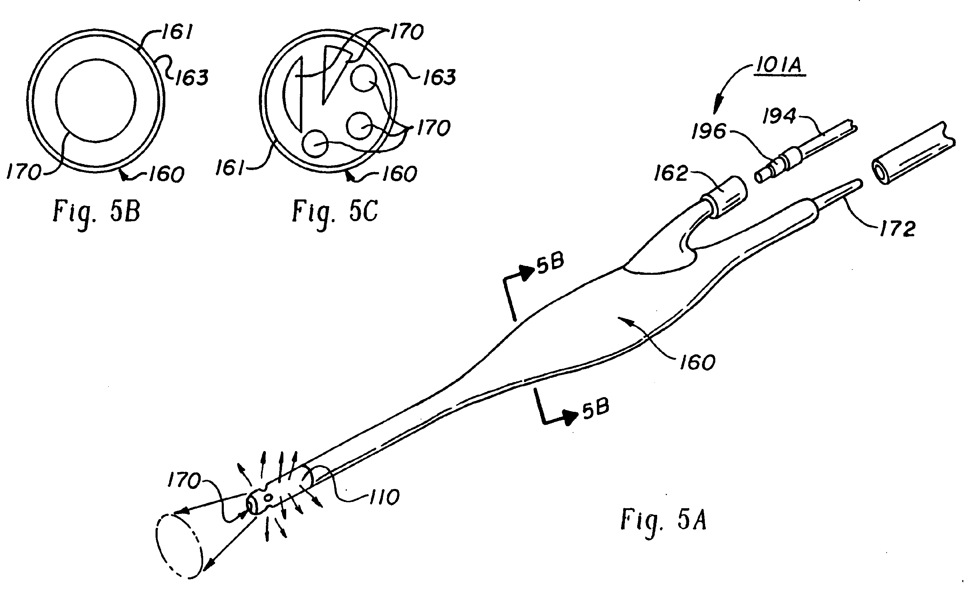

Referring now to Fig. 5A, there is shown a suction/blower device 101A. Device 101A is a surgical instrument typically used to remove material (e.g., fluid or tissue) from a surgeons field of view. In this respect, device 101A suctions or blows the obscuring material. Device 101A is generally comprised of a light emitter 110, a light distributor 160 and air passageway(s) 170. Light distributor 160 includes a connecting member 162 dimensioned to receive a mating connecting member 196 from cable 194. Cable 194 is connected to a light source (not shown).

It is important to note that light distributor 160 not only carries light to light emitter 110, but also provides a support structure for suction/blower device 101A. In this respect, light distributor 160 includes a light distribution member 161, which is constructed of a rigid material and formed into a suitable shape for a user to conveniently hold device 101A. Light distribution member 161 transmits light and defines passageway(s) 170. Passageway(s) 170 are generally tubular hollow channels formed along the length of light distributor 160. Figs. 5B and 5C illustrate two different embodiments for light distributor 160. Passageway(s) 170 provides a conduit for air, or other gas or fluid. Light distributor 160 also includes an outer layer 163. Outer layer 163 may take the form of a heat-shrinked film, coating or tubing. Outer layer 163 provides a protective layer for light distribution member 161. Similarly, an inner layer (not shown) may line the inner surface of light distribution member 161. The outer and inner layers protect the internal light propagation from impairment (e.g., blood or other materials that can cause light loss). It should be appreciated that light distributor 160 may be constructed of a plurality of walls of varying thickness. The walls may take the form of a film, coating or tubing. Moreover, the film, coating or tubing may extend along the full length of light distributor 160, or only along a portion thereof.

A connector 172 is provided to receive a mating connector from a hose 174. Hose 174 is connected to a vacuum generating means (not shown), where device 101A is used for suction, or is connected to a blower means (not shown), where device 101A is used for blowing. Light emitter 110 is located at the tip end of device 101A, and surrounds passageway(s) 170. Light emitter 110 is suitably formed to provide diffuse light in directions transverse to the longitudinal axis of device 101A, and to provide direct light in a direction generally parallel to the longitudinal axis of device 101A. In this way, the direct light provides maximum illumination on the material being suctioned or blown. At the same time, the diffuse light provides sufficient, but not over bright, illumination of the area surrounding the material being suctioned or blown. As a result, the user's vision of the material being suctioned or blown is not impaired.

It should be appreciated that light distributor 160 and light emitter 110 form an integral part of the suction/blowing device 101A, and thus eliminate the need for an external lighting device mounted to the suction/blowing device, a lighting device mounted elsewhere in an operating room, or a hand held lighting device.

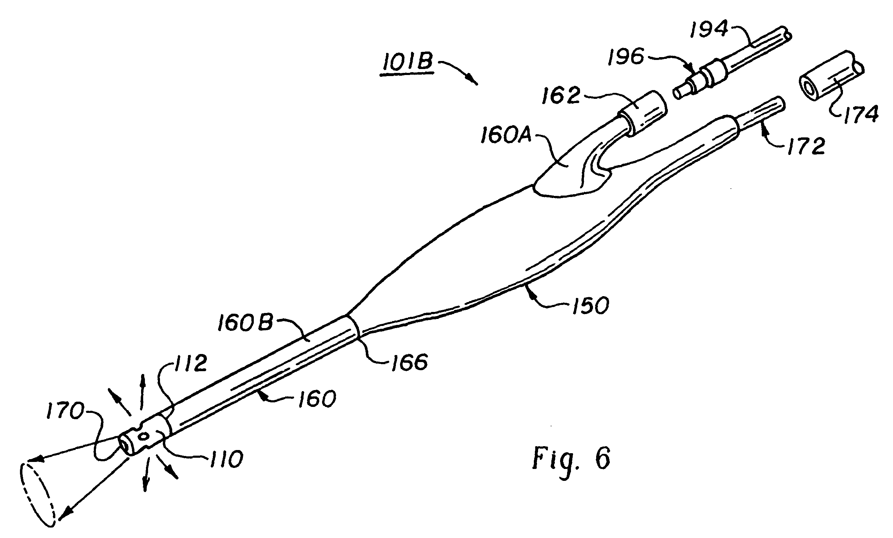

Fig. 6 illustrates an alternative embodiment of suction/blower device 101A. Suction/blower device 101B is similar in many respects to suction/blower device 101A ; however, light emitter 110 and light distributor 160 are disposable in this embodiment. In this respect, suction/blower 101B is generally comprised of a light emitter 110, a rigid body member 150, a light distributor 160 having a fixed portion 160A and a detachable portion 160B, and a tube 170. Body member 150 is constructed of a rigid material (e.g., plastic) and formed into a suitable shape for a user to conveniently hold device 101B. Body member 150 surrounds fixed portion 160A of light distributor 160. Fixed portion 160A includes a connecting member 162. Fixed portion 160A and detachable portion 160B are connected at interface 166. A hollow channel is formed along the length of portions 160A and 160B to provide tube 170. Light emitter 110 is optionally detachable from light distributor 160 at interface 166.

It should be appreciated that suction/blower device 101B has the advantage of having a detachable light emitter 110 and light distributor 160. This allows for convenient replacement of the portions of device 101 B which may require sterilization. As a result, only an inexpensive and small portion of device 101B is disposed, thus saving the expense of replacing the entire suction/blower device 101B.

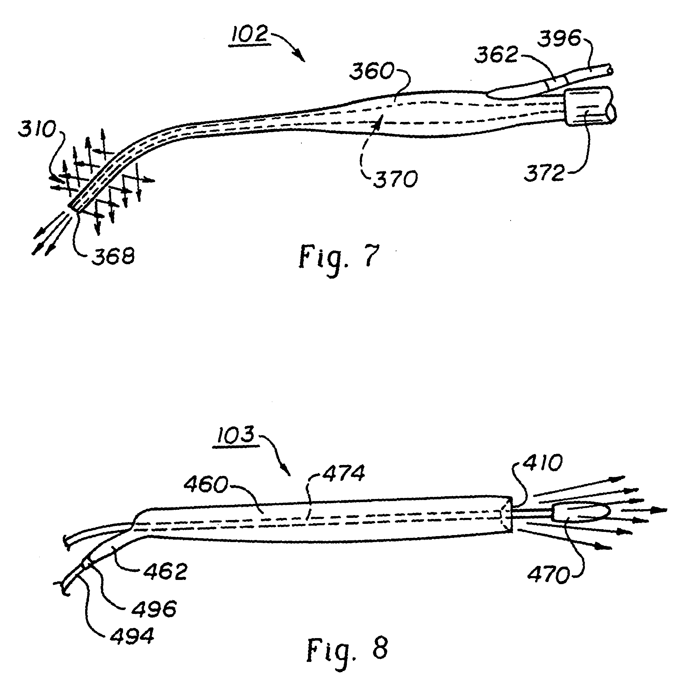

Fig. 7 illustrates another suction/blower device 102. Device 102 is generally comprised of a light emitter 310, a light distributor 360 and a tube 370. Light distributor 360 has a connecting member 362 dimensioned to receive a mating connecting member 396 from cable 394. Cable 394 is connected to a light source (not shown). It is important to note that light distributor 360 not only carries light to light emitter 310, but also provides a support structure for suction/blower device 102. In this respect, light distributor 360 is constructed of a rigid material and formed into a suitable shape for a user to conveniently hold device 102. In addition, a hollow channel is formed along the length of light distributor 360 to provide tube 370. Light distributor 360 is preferably formed of an inexpensive plastic material. Tube 370 includes a connector 372, dimensioned to receive a mating connector from a hose 374. Hose 374 is connected to a vacuum generating means (not shown), where device 102 is used for suction, or is connected to a blower means (not shown), where device 102 is used for blowing. Light emitter 310 is located at tip 368 of light distributor 360, and surrounds tube 370. Light emitter 310 is suitably formed to provide diffuse light in directions transverse to the longitudinal axis of tip 368, and to provide direct light in a direction generally parallel to the longitudinal axis of tip 368. In this way, the direct light provides maximum illumination on the material being suctioned or blown. At the same time, the diffuse light provides sufficient, but not over bright, illumination of the area surrounding the material being suctioned or blown. As a result, the user's vision of the material being suctioned or blown is not impaired.

It should be appreciated that light distributor 360 is easily and conveniently attached to and detached from cable 394 and hose 374. As a result, light delivery system 202 is easily replaced where sterilization is required.

Fig. 8 illustrates an electrosurgical pencil device 103. Electrosurgical pencil device 103 is used to destroy tissue by burning the tissue with a cauterizing tip. Device 103 is generally comprised of a light emitter 410, a light distributor 460 and a cauterizing tip 470. Light distributor 460 has a connecting member 462 dimensioned to receive a mating connecting member 496 from a cable 494. Cable 494 is connected to a light source (not shown). It is important to note that light distributor 460 not only conducts light to light emitter 410, but also provides a support structure for device 103. In this respect, light distributor 460 is constructed of a rigid material and formed into a suitable shape for a user to conveniently hold device 103. In addition, a channel is formed along the length of light distributor 460 to provide a passageway for electrical conductor 474. Electrical conductor 474 connects to cauterizing tip 470, to provide power thereto. Light emitter 410 is suitably formed to provide diffuse light in directions transverse to the longitudinal axis of tip 470, and to provide direct light in a direction generally parallel to the longitudinal axis of tip 470. In this way, the direct light provides maximum illumination on the material being cauterized. At the same time, the diffuse light provides sufficient, but not over bright, illumination of the area surrounding the material being cauterized. As a result, the user's vision of the material being cauterized is not impaired.

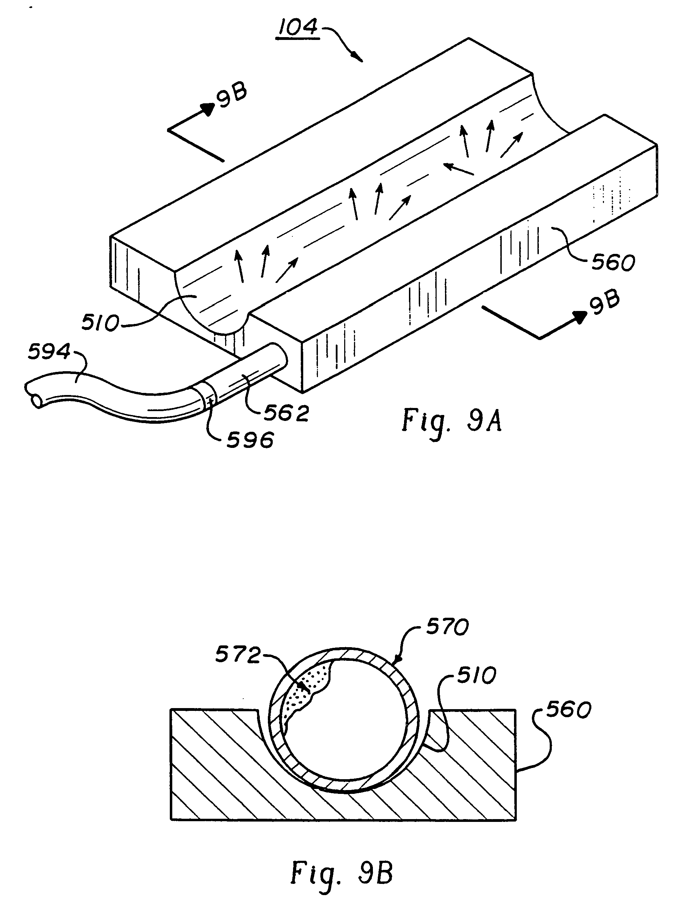

Referring now to Fig. 9A, there is shown a transillumination tray 104 for illuminating a bodily structure (e.g., vein, artery, finger, or small organ). Tray 104 is generally comprised of a light distributor 560 and a light emitter 510. Light distributor 560 includes a connecting member 562 dimensioned to receive a mating connecting member 596 from a cable 594. Cable 594 is connected to a light source (not shown). It is important to note that light distributor 560 not only conducts light to light emitter 510, but also provides a support base for tray 104. In this respect, light distributor 560 is constructed of a rigid material and formed into a suitable shape for receiving a generally U-shaped light emitter 510. Light emitter 510 is shaped to receive a bodily structure, and thoroughly illuminate it. In this respect, light is emitted in all directions from the surface of light emitter 510. Fig. 9B illustrates a cross-sectional view of tray 104 with a vein/artery 570 located on tray 104 for examination. Light emitter 510 illuminates an obstruction 572 in vein/artery 570.

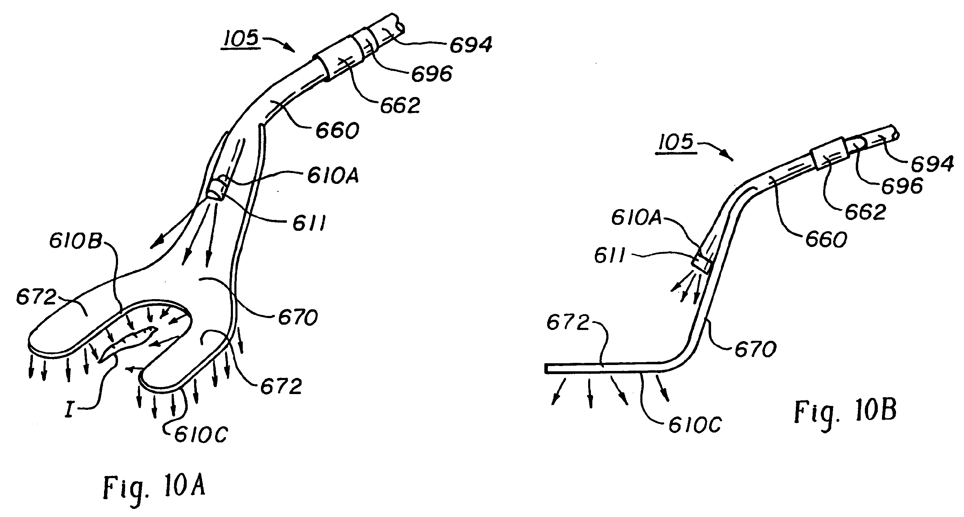

Figs. 10A and 10B show a stabilizer device 105. Stabilizer device 105 is generally comprised of light emitters 610A, 610B and 610C, and a light distributor 660. Light distributor 660 includes a central portion 670, arm portions 672, and connecting member 662. Connecting member 662 is dimensioned to receive a mating connecting member 696 from a cable 694 (such as a light pipe). Cable 694 is connected to a light source (not shown). It is important to note that light distributor 660 not only carries light to light emitters 610A, 610B and 610C, but also provides a support structure for stabilizer device 105. In this respect, light distributor 660 is constructed of a rigid material and formed into a suitable shape for a user to conveniently hold device 102. Light emitters 610A, 610B and 610C provide different lighting conditions. In this respect, light emitter 610A may includes a lens 611 for providing direct focused light on incision work area I. Light emitter 610B is formed along the periphery defined by central portion 670 and arm portions 672. Light emitter 610B provides indirect diffuse light for incision work area I. Light emitter 610C is formed along the lower edge (i.e., bottom) of central portion 670 and arm portions 672. Light emitter 610C may provide indirect diffuse light or glowing light for transillumination of a bodily structure.

It should be appreciated that in an alternative embodiment, stabilizer device 105 may be suitably arranged to attach (e.g., using a clip or other attachment means) to a metal stabilizer having the same general shape as stabilizer device 105. In this regard, the strength of the material forming stabilizer device 105 may not sufficient for a particular application. Accordingly, the metal stabilizer provides the desired strength.

Referring now to Fig. 11, there is shown retractor devices 106A, 106B and 106C for retracting body structure T (which may include, bodily tissue, bone, organs or the like). Retractor device 106A is comprised of a retractor member 770A and a light delivery system 702A. Retractor member 770A includes a horizontal portion 772, a vertical portion 774, and a support member 776. Support member 776 is arranged between horizontal portion 772 and a rigid mount (not shown). Light delivery system 702A is mounted to the front face of vertical portion 774, and includes a light distributor 760A and a light emitter 710A. Light distributor 760A bends to follow the general shape of retractor member 770A, and receives light from a light source (not shown). A suitable adhesive may be used to attach light delivery system 702A to vertical portion 774. Light emitter 710A provides diffuse or directional light into the work area.

Retractor device 106B is generally comprised of a retractor member 770B and a light delivery system 702B. Retractor member 770B is a rake retractor having a plurality of prongs. Light delivery system 702B includes an attachment member 780B, light distributor 760B, and light emitter 710B. Attachment member 780B has engagement means 784B for attaching light delivery system 702B to retractor member 770B. Light distributor 760B receives light from a light source (not shown). Light emitter 710B includes a top portion 711B and a side portion 713B. Light emitter 710B provides diffuse or directional light into the work area.

Retractor device 106C is a rake retractor formed of a translucent material (e.g., plastic). Retractor device 106C includes light distributor 760B and light emitter 710C. The light distributor 760B and light emitter 710C form the structural member of retractor device 106C.

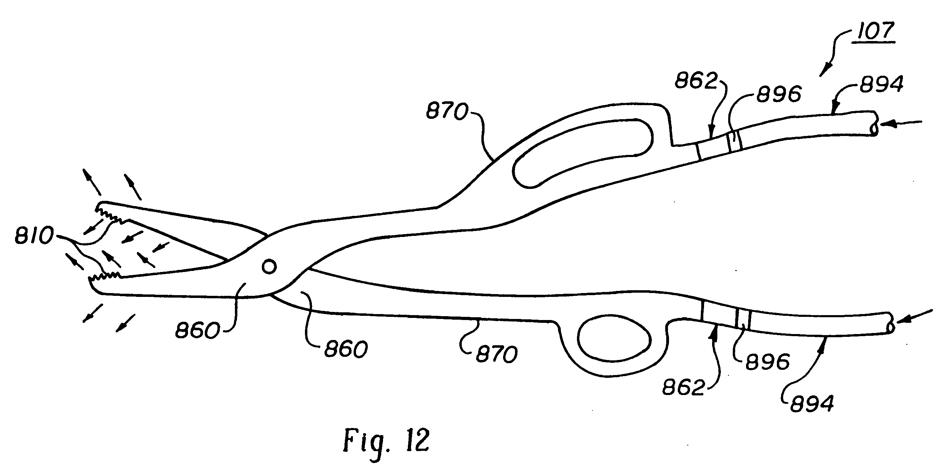

Referring now to Fig. 12, there is shown an illuminated forceps 107 having an integrated light delivery system. Forceps 107 is generally comprised of light distributors 860 and light emitters 810. Each light distributor 860 includes a pair of arms 870 and a pair of connecting members 862. Connecting members 862 connect to a mating connecting members 896 of light source cables 894. Cables 894 connect to a light source (not shown). Light emitters 810 forms the gripping surfaces of arms 870, and provide focused or diffuse light. It should be appreciated that light emitters 810 may provide light for inspection, as well as transillumination. In the case of inspection the light is used to inspect a work area before proceeding with a further operation. With regard to transillumination, the light may be use to examine a bodily structure. For instance, a vein may be transilluminated to identify a blood clot before clamping and cutting.

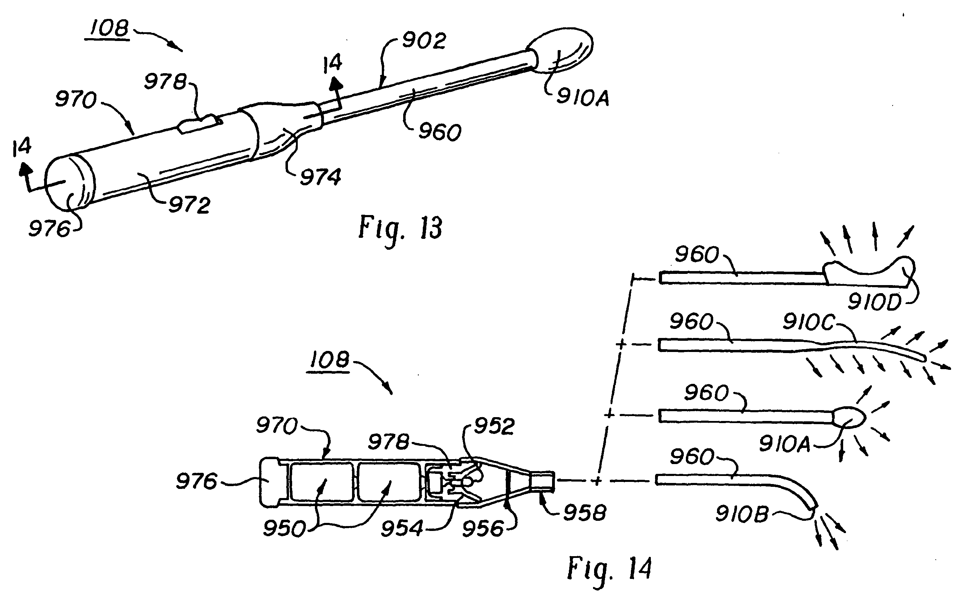

Figs. 13 and 14 show a multi-purpose lighting device 108. Device 108 is generally comprised of a light delivery portion 902 and a handle portion 970. Light delivery portion 902 includes a light distributor 960 and a light emitter 910A. Handle portion 970 includes a central housing 972, a connecting member 974 and an endcap 976. As shown in Fig. 14, handle portion 970 houses a power source 950 (e.g., batteries), a light source 952 (e.g., light bulb), a reflector 954, a light filter 956 and a switch means 974. Reflector 954 reflects the light generated by light source 952. Light filter 956 filters the reflected light before it exits through the open end of connecting member 974. Light source 952 is turned on and off by switch means 978. It should be noted that endcap 976 may include a contact member for completing a circuit for powering light source 952.

It should be appreciated that connecting member 974 is dimensioned to receive a light distributor 960, as best seen in Fig. 14. Accordingly, a variety of different types of light delivery portions 902 can be used in combination with handle portion 970, wherein handle portion 970 provides a light source. For instance, light delivery portion 902 may include a light emitter 910A in the form of an illuminated ball (Fig. 13). The surface of the ball may be covered with cotton to form an illuminated cotton swab. Alternatively, light delivery portion 970 may include a light emitter 901B in the form of an end light (Fig. 14), a light emitter 910C in the form of an illuminated tongue depressor (Fig. 14), and a light emitter 910D in the form of a transillumination tray (Fig. 14), similar to tray 104, described above. Through the use of a variety of attachable light delivery portions 902, device 108 serves a wide range of functions.

It should be appreciated that the light delivery portions shown in Fig. 13 and 14 are shown solely for the purpose of illustration. In this respect, other types of light delivery portions, serving functions similar to those of the illustrated embodiments, are also contemplated. Moreover, it should be appreciated that the portable light source housed in the handle portion may be suitably replaced by a remote light source (e.g., see Fig. 4A), with a light pipe for conveying the light therefrom.

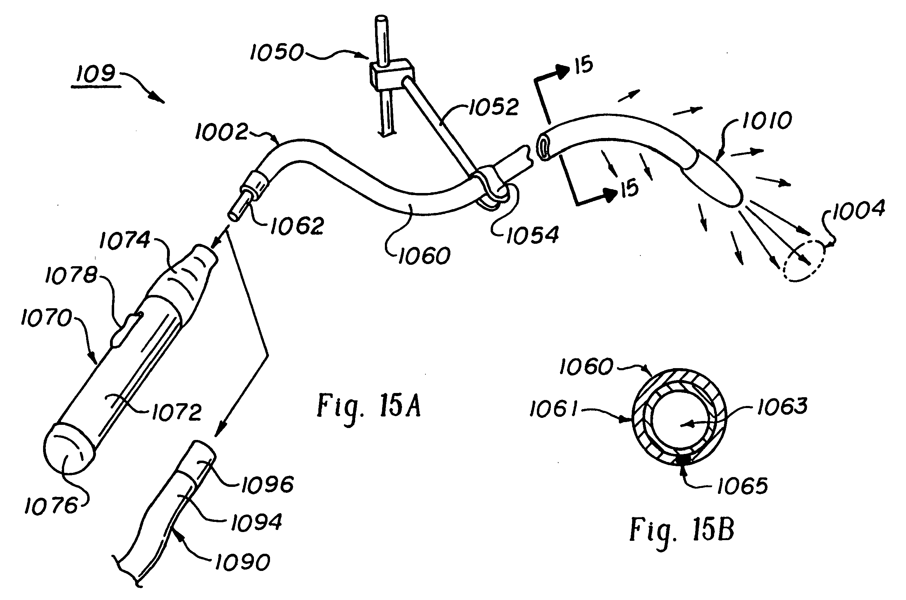

Referring now to Fig. 15A, there is shown a lighting device 109, which functions as a flexible and formable "trouble light." Lighting device 109 is generally comprised of a light delivery portion 1002 and a handle portion 1070. Light delivery portion 1002 includes a light distributor 1060 and a light emitter 1010. Light distributor 1060 includes a connecting member 1062 for connecting light distributor 1060 to handle portion 1070. It should be noted that in a preferred embodiment, light distributor 1060 is flexible. As seen in the cross-sectional view of Fig. 15B, light distributor 1060 is comprised of a light pipe member 1063, a translucent or colored outer sheath 1061 and a formable wire 1065. Formable wire 1065 allows light distributor 1060 to be bent or positioned in a suitable manner. Light emitter 1010 is detachable from light distributor 1060 to provide a variety of multi-purpose light emitters. In the embodiment shown in Fig. 15A, light emitter 1010 takes the form of a glowing tip, which is rotatable to alter the focus, size or light intensity of lighted area 1004.

Handle portion 1070 is similar to handle portion 970, described above. In this regard, handle portion 1070 includes a central housing 1072, connecting member 1074, endcap 1076, and a switch means 1078. Handle portion 1070 houses a light source and a power source. It should be appreciated that handle portion 1070 is suitably replaced by a light pipe 1090 of conventional light source. Light pipe 1090 includes a cable 1094 and a mating connecting member 1096, which mates with connecting member 1062.

Device 109 may optionally include a rigid support member 1050 to keep light distributor 1060 from changing positions. Support member 1050 includes an arm 1052 and clamp 1054. Clamp 1054 engages with light distributor 1060.

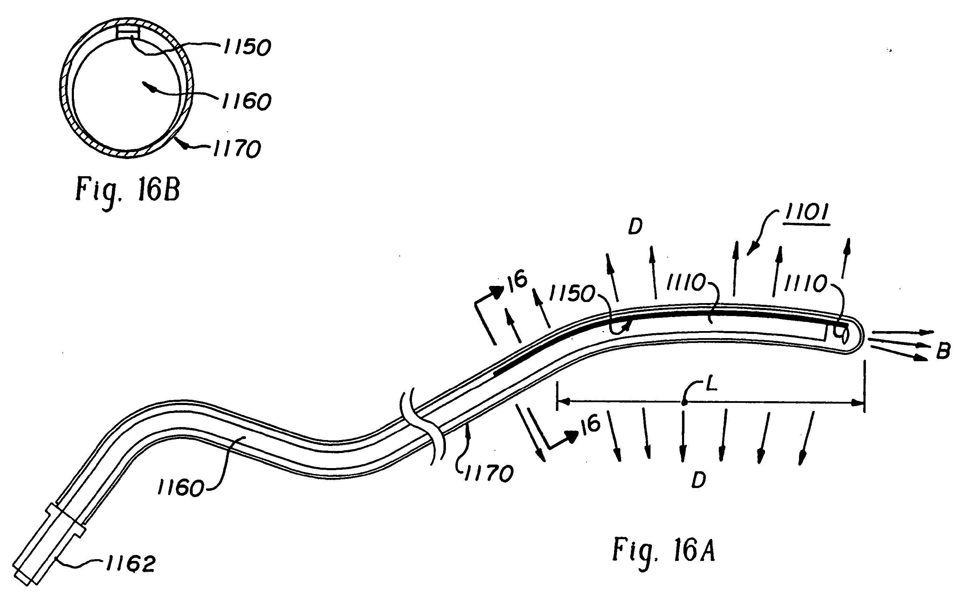

Referring now to Fig. 16A, there is shown a formable "rope"' lighting device 1101, which is similar to the lighting device shown in Figs. 15A and 15B. Lighting device 1101 is generally comprised of a light distributor 1160 and light emitters 1110. Light distributor 1160 includes a connecting member 1162 for connecting light distributor 1160 to a light source (not shown). It should be noted that in a preferred embodiment, light distributor 1160 is formed of a flexible optic light guide. As seen in the cross-sectional view of Fig. 16B, a protective outer sleeve 1170 covers light distributor 1160. Outer sleeve 1170 is preferably formed of a translucent or transparent material. An optional formable wire 1150 extends between light distributor 1160 and outer sleeve 1170, to permit lighting device 1101 to hold its shape once bent to a suitable position. Light emitters 1110 provide diffuse light D along length L, in addition to a focused beam of light B at the free end of lighting device 1101. It should be noted that at optional lens may be provided at the free end of lighting device 1101 to focus light B from light emitters 1110 in a desired pattern.

Referring now to Fig. 17, there is shown a trans-illuminating pickup or forceps 1102 having an attachable light delivery system 1200. Arrows A illustrate the direction in which forceps 1102 is movable. Light delivery system 1200 is generally comprised of a light distributor 1260 and a light emitter 1210. Light distributor 1260 includes connecting members (not shown) for connecting light delivery system 1200 to a light source (not shown). Light distributor 1260 preferably takes the form of an optic light guide cable, which may be either rigid or flexible. Attachment members 1280 connect light distributor 1260 to forceps 1102. In a preferred embodiment attachment members take the form of clips. An opening 1270 is formed at the tip end of one arm of forceps 1102. Opening 1270 is dimensioned to receive light emitter 1210. Light emitter 1210 provide light along length L. It should be appreciated that a second opening 1270 may be formed in the second arm of forceps 1102, in order to receive a second light emitter.

Referring now to Fig. 18, there is shown a trans-illuminating retractor 1103 having an attachable light delivery system 1300. Arrows A illustrate the directions in which retractor 1103 is movable. Light delivery system 1300 is generally comprised of a light distributor 1360 and a light emitter 1310. Light, distributor 1360 includes connecting members (not shown) for connecting light delivery system 1300 to a light source (not shown). Light distributor 1360 preferably takes the form of an optic light guide cable, which may be either rigid or flexible. A connector 1364 is provided to connect and interface light distributor 1360 with light emitter 1310. Attachment members 1380 and 1388 connect light delivery system 1300 to forceps 1103. In a preferred embodiment of the present invention attachment member 1380 takes the form of a clip. Light emitter 1310 extends along the inner surface of the retractor arms.

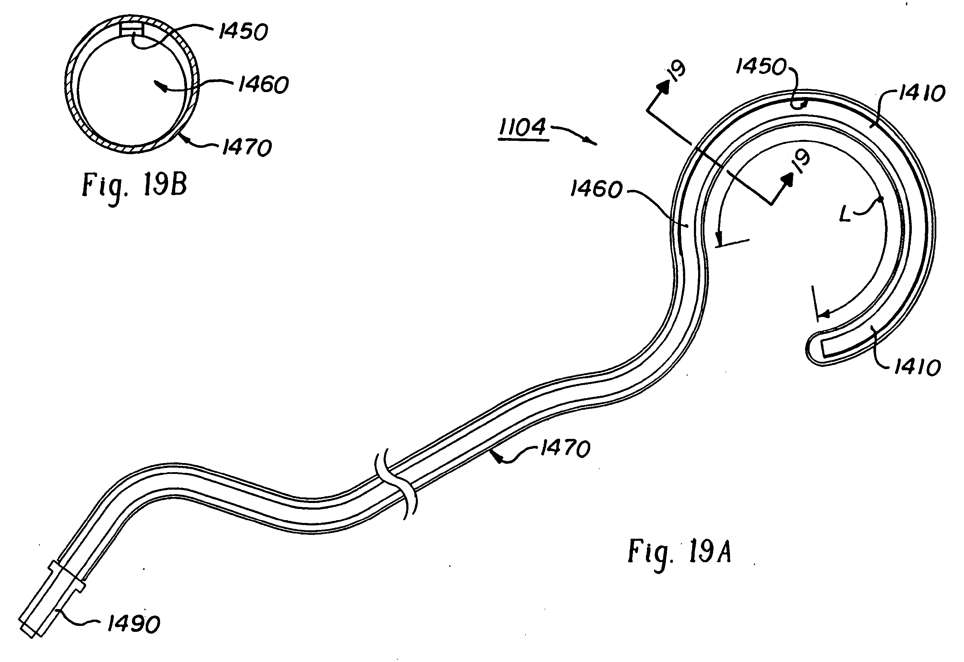

Figs. 19A and 19B illustrate a spring-formed "rope" lighting device 1104. lighting device 1104 is generally comprised of a light distributor 1460 and a light emitter 1410. Light distributor 1460 interfaces with a self-contained miniature light source unit 1490. Light source unit 1490 includes a light source (e.g., LED, incandescent light, laser diodes or the like) and a power source (e.g., a button battery cell or the like). The miniaturization and portability of light source unit 1490 allows lighting device 1104 to be arrangeable within a bodily structure, such as abody cavity. Alternatively, a remote light source may substitute for self-contained light source unit 1490. It should be noted that in a preferred embodiment light distributor 1460 is formed of a flexible optic light guide. As best seen in the cross-sectional view of Fig.19B, a protective outer sleeve 1470 covers light distributor 1460. Outer sleeve 1470 is preferably formed of a translucent or transparent material. A spring 1450 extends between light distributor 1460 and outer sleeve 1470. Spring 1450 may be formed of a material which allows it to return to its original shape after being compressed. Accordingly, spring 1450 has a "memory," which allows for advantageous use of lighting device 1104, as will be described below. Light emitter 1410 provides diffuse light D along length L.

It should be appreciated that while lighting device 1104 is shown with a generally round cross-sectional area, lighting device 1104 may have a cross-sectional area of other shapes, including a square and octagon.

Lighting device 1104 finds particularly advantageous use as a means for holding a cavity open during a surgical procedure. In this regard, lighting device 1104 is compressed (i.e., squeezed) and inserted through an opening into a cavity (e.g., a heart chamber). When the compressive force is removed from lighting device 1104 the "memory" of spring 1450 causes the device to return to its original shape (i.e., spring open). As a result, the cavity opening is conveniently held open during further surgical procedures. It should be appreciated that spring 1450 may be suitably shaped to fit a particular application.

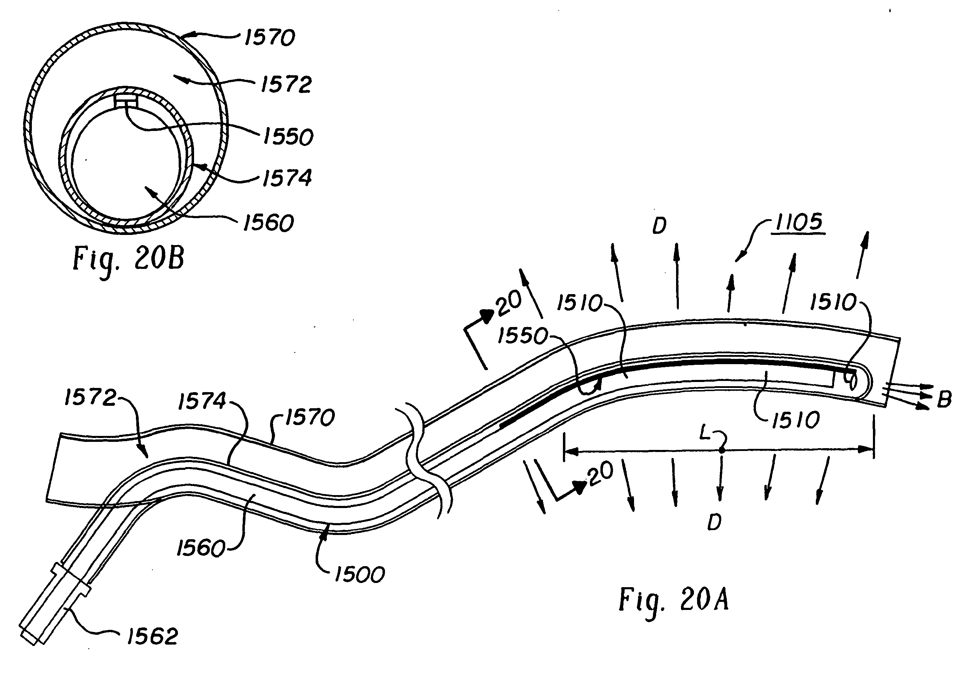

Figs. 20A and 20B illustrates a smoke evacuation tube 1105 having an integrated light delivery system 1500. Light delivery system 1500 is generally comprised of a light distributor 1560 and light emitters 1510. Light distributor 1560 includes a connecting member 1562 for connecting light distributor 1560 to a light source (not shown). Light distributor 1560 is preferably formed of a flexible optic light guide. As best seen in the cross-sectional view of Fig. 20B, a protective outer sleeve 1574 covers light distributor 1560. Outer sleeve 1574 is preferably formed of a translucent or transparent material. An optional formable wire 1550 extends between light distributor 1560 and outer sleeve 1574, to allow smoke evacuation tube 1105 to hold its shape once arranged in a desired position. Light emitters 1510 provide diffuse light D along length L, in addition to a beam of light B. It should be noted that an optional lens may be provided at the free end of smoke evacuation tube 1105 to focus light B from light emitter 1510 in a desired pattern.

A hollow tube 1570 forms an evacuation chamber 1572 for removing smoke. As best seen in Fig. 20B, hollow tube 1570 surrounds and connects to outer sleeve 1574. Hollow tube 1570 is preferably formed of a translucent or transparent material. It should be appreciated that in an alternative embodiment, sleeve 1574 and tube 1570 are suitably arranged adjacent to each other.

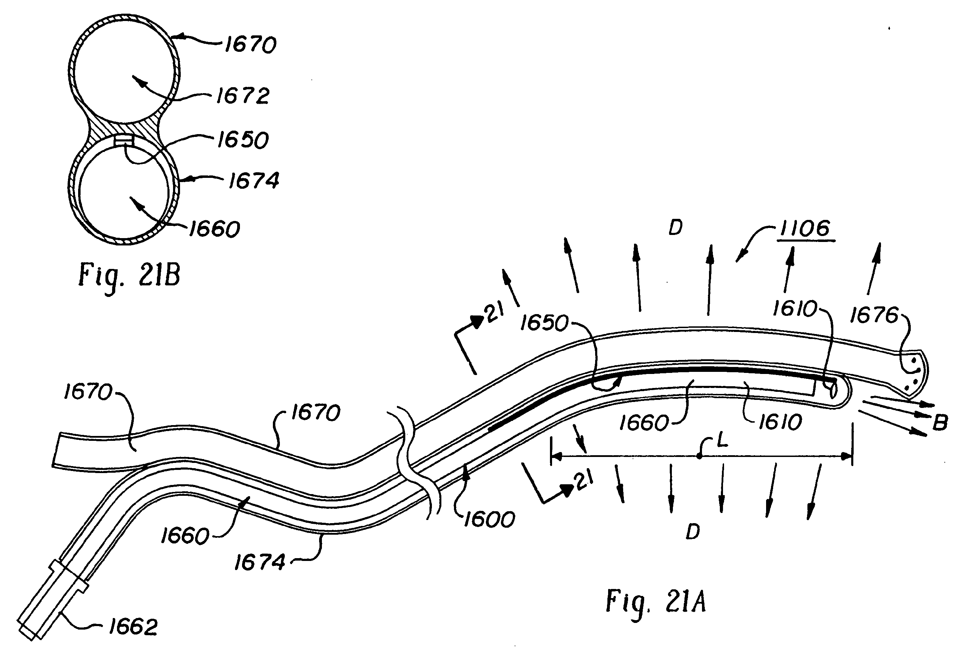

Figs. 21A and 21B illustrates a suction tube 1106 having an integrated light delivery system 1600. Light delivery system 1600 is generally comprised of a light distributor 1660 and light emitters 1610. Light distributor 1660 includes a connecting member 1662 for connecting light distributor 1660 to a light source (not shown). Light distributor 1660 is preferably formed of a flexible optic light guide. As best seen in the cross-sectional view of Fig. 21B, a protective outer sleeve 1674 covers light distributor 1660. Outer sleeve 1674 is preferably formed of a translucent or transparent material. An optional formable wire 1650 extends between light distributor 1660 and outer sleeve 1674, to permit suction tube 1106 to hold its shape once arranged in a desired position. Light emitters 1610 provide diffuse light D along length L, in addition to a focused beam of light B. It should be noted that an optional lens may be provided at the free end of suction tube 1106 to focus light B from light emitter 1610 in a desired pattern. A hollow tube 1670 forms a suction chamber 1672 for suctioning smoke and other materials. A nozzle 1676 is formed at the free end of hollow tube 1670. As best seen in Fig. 21B, hollow tube 1670 is arranged adjacent and connected to outer sleeve 1674. Hollow tube 1670 is preferably formed of a translucent or transparent material.

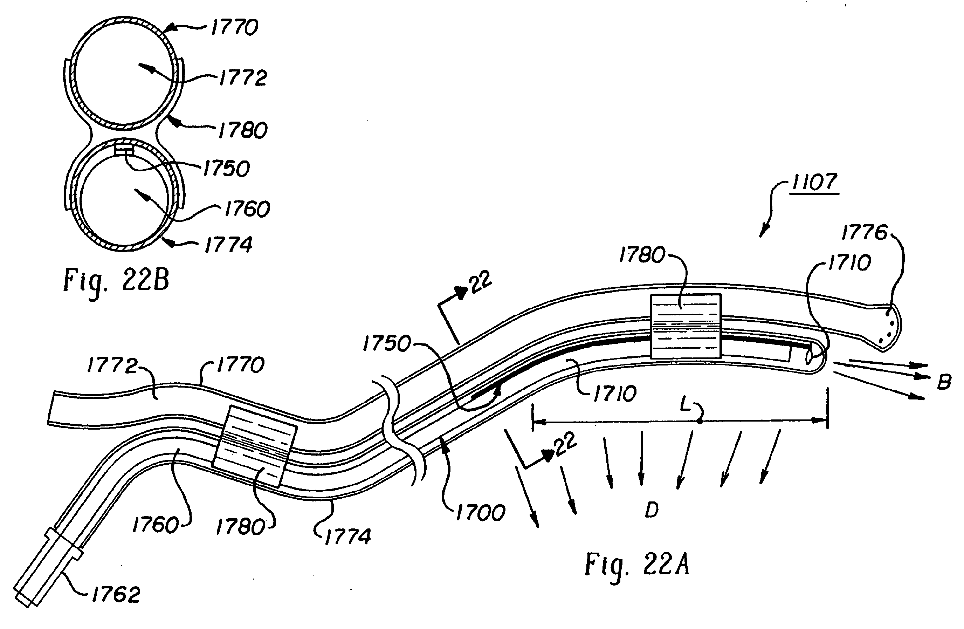

Figs. 22A and 22B illustrates a suction tube 1107 having an attachable light delivery system 1700. Light delivery system 1700 is generally comprised of a light distributor 1760 and light emitters 1710. Light distributor 1760 includes a connecting member 1762 for connecting light distributor 1660 to a light source (not shown). Light distributor 1760 is preferably formed of a flexible optic light guide. As best seen in the cross-sectional view of Fig. 22B, a protective outer sleeve 1774 covers light distributor 1760. Outer sleeve 1774 is preferably formed of a translucent or transparent material. An optional formable wire 1750 extends between light distributor 1760 and outer sleeve 1774, to permit suction tube 1107 to hold its shape once arranged in a desired position. Light emitters 1710 provide diffuse light D along length L, in addition to a beam of light B. It should be noted that an optional lens may be provided at the free end of suction tube 1107 to focus light B from light emitter 1710 in a desired pattern.

A hollow tube 1770 forms a suction chamber 1772 for suctioning smoke and other materials. A nozzle 1776 is formed at the free end of hollow tube 1670. Hollow tube 1770 is preferably formed of a translucent or transparent material. Attachment members 1780 connect hollow tube 1770 to outer sleeve 1774. In a preferred embodiment, attachment member 1780 takes the form of a clip having a pair of gripping members respectively dimensioned to receive hollow tube 1770 and sleeve 1774 (Fig. 22A). However, it should be appreciated that attachment member 1780 may take other suitable forms.

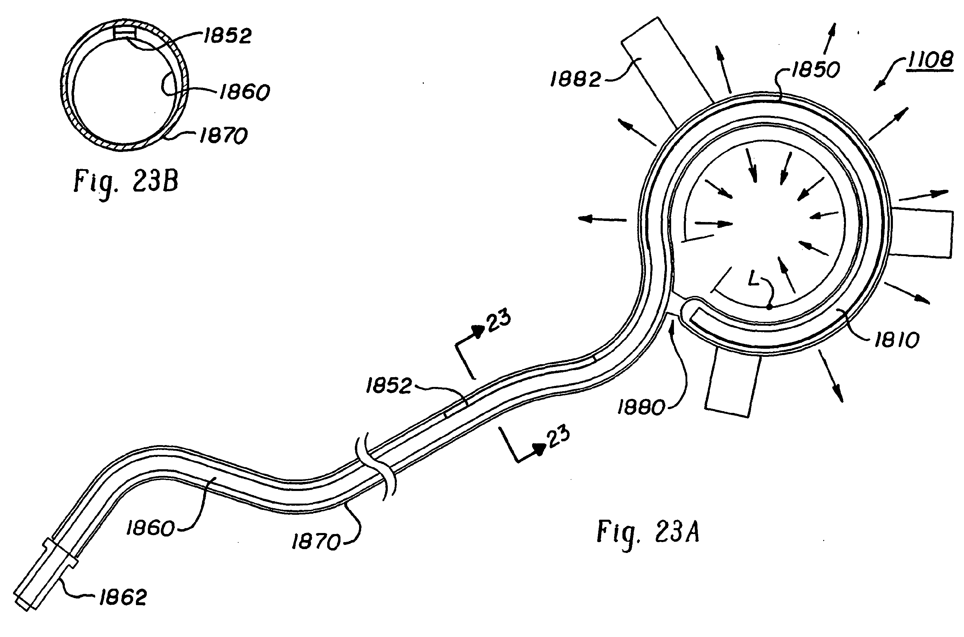

Referring now to Fig. 23A, there is shown a ring-shaped "rope" lighting device 1108. Lighting device 1108 is generally comprised of a light distributor 1860 and light emitters 1810. Light distributor 1860 includes a connecting member 1862 for connecting light distributor 1860 to a light source (not shown). It should be noted that in a preferred embodiment of the present invention, light distributor 1860 is formed of a flexible optic light guide. As seen in the cross-sectional view of Fig. 23B, a protective outer sleeve 1870 covers light distributor 1860. Outer sleeve 1870 is preferably formed of a translucent or transparent material. A custom-formed spring temper wire 1850 extends between light distributor 1860 and outer sleeve 1870. Wire 1850 may be compressed and will return to its original shape. Light emitter 1810 provides light along length L. A fastener 1880 is provided to hold lighting device 1108 in a desired shape. Fastener 1880 may take many suitable forms, including a mechanical fastener or adhesive (e.g., glue). A secondary wire 1852 is provided along a portion of light distributor 1860. Wire 1852 may be malleable or spring temper. Tabs 1882 hold lighting device 1108 in a desired location, and can also be used to retract tissue during a surgical procedure. In a preferred embodiment, tabs 1882 take the form of adhesive tape.

It is important to note that the present invention as illustrated by the embodiments of Figures 11 and 18 allows for significant improvements to surgical procedures. In this regard the light delivery system attaches to a surgical instrument or forms an integral part of the structure of a surgical instrument. Accordingly, the present invention eliminates the need for a lighting device mounted elsewhere in an operating room, or a hand held lighting device. Moreover, the light emitter of the present invention provides light in a suitable direction, and with varying intensity levels. As a result, the medical personnel receive an excellent view of the work area.

The invention has been described with reference to a preferred embodiment shown in Figures 11 and 18. Obviously, modifications and alterations will occur to others upon a reading and understanding of this specification. In this regard, it should be appreciated that the present application discloses numerous exemplary embodiments for the purpose of illustrating the present invention.

## Claims

1. An instrument (1103, 106A to C ) for aiding a surgical procedure comprising a handle and a retractor arm having one end connected to the handle and another end remote from the one end, **characterized by** an elongated light emitter (1310, 710A to C) extending along the length of the retractor arm, and a connector (1364) for connecting the light emitter (1310, 710A to C ) to a light distributor (1360), the light emitter (1310, 710A to C) having light extracting formations (22, 28, 30, 32) along at least a portion of the length of the light emitter.

2. The instrument of claim 1 further **characterized in that** the light emitter (1310, 710A to C) is substantially transparent.

3. The instrument of claim 1 or claim 2 further **characterized in that** the light emitter (1310, 710A) is oriented at an angle with respect to the handle.

4. The instrument of any one of the preceding claims further **characterized in that** the retractor arm is oriented at an angle with respect to the handle.

5. The instrument of any of the preceding claims further **characterized in that** the light extracting formations comprise a printed pattern (22) on a surface (20) of the light emitter (1310, 710A to C).

6. The instrument of any one of the preceding claims further **characterized by** attachment means (1380, 780B) for attaching the light emitter (1310, 710B) to the retractor arm such that the light emitter (1310, 710B) and retractor arm are substantially parallel.

7. The instrument of claim 6 further **characterized in that** the attachment means (1380) comprises clips.

8. The instrument of any one of the preceding claims further **characterized in that** the retractor arm includes a generally curved cross-section.

9. The instrument of any one of the preceding claims further **characterized in that** the light distributor (1360) comprises an optic light guide cable.

10. The instrument of claim 9 further **characterized in that** the cable (1360) is rigid.

11. The instrument of claim 9 further **characterized in that** the cable (1360) is flexible.

12. The instrument of any one of the preceding claims further **characterized by** clips (1380) for attaching the light emitter (1310) to the retractor arm.

13. The instrument of any one of the preceding claims further **characterized in that** the light extracting formations (22, 28, 30, 32) are along substantially the entire length of the light emitter (1310, 710A to C).

## Patentansprüche

1. Instrument (1103, 106 A bis C) zum Unterstützen einer chirurgischen Operation mit einem Handgriff und einem Retraktorarm, dessen eines Ende mit dem Handgriff verbunden ist und dessen anderes Ende von dem anderen Ende entfernt ist, **gekennzeichnet durch** einen länglichen Lichtemitter (1310, 710 A-C), der sich entlang der Länge des Retraktorarms erstreckt, und einen Verbinder (1364) zum Verbinden des Lichtemitters (1310, 710 A-C) mit einem Lichtverteiler (1360), welcher Lichtemitter (1310, 710 A-C) Lichtextraktionsgebilde (22, 28, 30, 32) entlang zumindest eines Teils der Länge des Lichtemitters aufweist.

2. Instrument nach Anspruch 1, weiterhin **dadurch gekennzeichnet, dass** der Lichtemitter (1310, 710 A-C) im wesentlichen transparent ist.

3. Instrument nach Anspruch 1 oder Anspruch 2, weiterhin **dadurch gekennzeichnet, dass** der Lichtemitter (1310, 710 A) unter einem Winkel mit Bezug auf den Handgriff orientiert ist.

4. Instrument nach einem der vorhergehenden Ansprüche, weiterhin **dadurch gekennzeichnet, dass** der Retraktorarm unter einem Winkel mit Bezug auf den Handgriff orientiert ist.

5. Instrument nach einem der vorhergehenden Ansprüche, weiterhin **dadurch gekennzeichnet, dass** die Lichtextraktionsgebilde ein gedrucktes Muster (22) auf einer Oberfläche (20) des Lichtemitters (1310, 710 A-C) aufweisen.

6. Instrument nach einem der vorhergehenden Ansprüche, weiterhin **gekennzeichnet durch** Befestigungsmittel (1380, 780B) zum Befestigen des Lichtemitters (1310, 710B) an dem Retraktorarm in der Weise, dass der Lichtemitter (1310, 710B) und der Retraktorarm im wesentlichen parallel sind.

7. Instrument nach Anspruch 6, weiterhin **dadurch gekennzeichnet, dass** die Befestigungsmittel (1380) Klemmen aufweisen.

8. Instrument nach einem der vorhergehenden Ansprüche, weiterhin **dadurch gekennzeichnet, dass** der Retraktorarm einen im allgemeinen gekrümmten Querschnitt enthält.

9. Instrument nach einem der vorhergehenden Ansprüche, weiterhin **dadurch gekennzeichnet, dass** der Lichtverteiler (1360) ein optisches Lichtleiterkabel aufweist.

10. Instrument nach Anspruch 9, weiterhin **dadurch gekennzeichnet, dass** das Kabel (1360) starr ist.

11. Instrument nach Anspruch 9, weiterhin **dadurch gekennzeichnet, dass** das Kabel (1360) flexibel ist.

12. Instrument nach einem der vorhergehenden Ansprüche, weiterhin **gekennzeichnet durch** Klemmen (1380) zum Befestigen des Lichtemitters (1310) an dem Retraktorarm.

13. Instrument nach einem der vorhergehenden Ansprüche, weiterhin **dadurch gekennzeichnet, dass** die Lichtextraktionsgebilde (22, 28, 30, 32) entlang im wesentlichen der gesamten Länge des Lichtemitters (1310, 710 A-C) angeordnet sind.

## Revendications

1. Instrument (1103, 106A à C) destiné à contribuer à un mode opératoire chirurgical comprenant une poignée et un bras d'écarteur ayant une extrémité raccordée à la poignée et une autre extrémité distante de l'une extrémité, **caractérisé par** un dispositif d'émission de lumière allongé (1310, 710A à C) s'étendant le long de la longueur du bras d'écarteur, et un dispositif de raccord (1364) destiné à raccorder le dispositif d'émission de lumière (1310, 710A à C) à un répartiteur de lumière (1360), le dispositif d'émission de lumière (1310, 710A à C) ayant des formations d'extraction de lumière (22, 28, 30, 32) le long d'au moins une portion de la longueur du dispositif d'émission de lumière.

2. Instrument selon la revendication 1, **caractérisé en outre en ce que** le dispositif d'émission de lumière (1310, 710A à C) est sensiblement transparent.

3. Instrument selon la revendication 1 ou 2 **caractérisé en outre en ce que** le dispositif d'émission de lumière (1310, 710A) est orienté selon un angle par rapport à la poignée.

4. Instrument selon l'une quelconque des revendications précédentes, **caractérisé en outre en ce que** le bras d'écarteur est orienté selon un angle par rapport à la poignée.

5. Instrument selon l'une quelconque des revendications précédentes, **caractérisé en outre en ce que** les formations d'extraction de lumière comprennent un motif imprimé (22) sur une surface (20) du dispositif d'émission de lumière (1310, 710A à C).

6. Instrument selon l'une quelconque des revendications précédentes, **caractérisé en outre par** un moyen d'attache (1380), 780B) destiné à attacher le dispositif d'émission de lumière (1310, 710B) au bras d'écarteur de telle manière que le dispositif d'émission de lumière (1310, 710B) et le bras d'écarteur soient sensiblement parallèles.

7. Instrument selon la revendication 6, **caractérisé en outre en ce que** le moyen d'attache (1380) comprend des pinces.

8. Instrument selon l'une quelconque des revendications précédentes, **caractérisé en outre en ce que** le bras d'écarteur comprend une section transversale généralement courbée.

9. Instrument selon l'une quelconque des revendications précédentes, **caractérisé en outre en ce que** le répartiteur de lumière (1360) comprend un câble optique guide de lumière.

10. Instrument selon la revendication 9, **caractérisé en outre en ce que** le câble (1360) est rigide.

11. Instrument selon la revendication 9, **caractérisé en outre en ce que** le câble (1360) est souple.

12. Instrument selon l'une quelconque des revendications précédentes, **caractérisé en outre par** des pinces (1380) destinées à attacher le dispositif d'émission de lumière (1310) au bras d'écarteur.

13. Instrument selon l'une quelconque des revendications précédentes, **caractérisé en outre en ce que** les formations d'extraction de lumière (22, 28, 30, 32) sont sensiblement le long de la longueur entière du dispositif d'émission de lumière (1310, 710A à C).
